# EUROPEAN PATENT SPECIFICATION

(11) **EP 4 196 061 B1**
(45) Date of publication and mention of the grant of the patent: **27.08.2025**
(21) Application number: 21749310.5
(22) Date of filing: 26.07.2021
(51) Int. Cl.: A61F 13/00

(54) **OFFLOADING ACCESSORY FOR USE WITH NEGATIVE-PRESSURE WOUND THERAPY DRESSINGS AND SYSTEMS**
ENTLASTUNGSZUBEHÖR ZUR VERWENDUNG MIT UNTERDRUCKWUNDTHERAPIEVERBÄNDEN UND -SYSTEMEN
ACCESSOIRE DE DÉCHARGEMENT DESTINÉ À ÊTRE UTILISÉ AVEC DES PANSEMENTS ET DES SYSTÈMES DE THÉRAPIE DE PLAIE À PRESSION NÉGATIVE

(30) Priority: 13.08.2020 US 202063065067 P
(43) Date of publication of application: 21.06.2023
(73) Proprietor: KCI Manufacturing Unlimited Company, Athlone, Co. Westmeath, N37XF22 (IE)
(72) Inventor: PRATT, Benjamin Andrew, Dublin Road Athlone, Co. Westmeath, N37XF22 (IE); RICE, Justin, Dublin Road Athlone, Co. Westmeath, N37XF22 (IE)
(74) Representative: Simmons & Simmons
(86) International application number: PCT/IB2021/056736
(87) International publication number: WO 2022/034408

(56) References cited:
- WO-A1-2019/084006
- WO-A1-2019/177683
- WO-A1-2020/040917

## Description

### TECHNICAL FIELD

The invention set forth in the appended claims relates generally to tissue treatment systems and more particularly, but without limitation, to dressings for tissue treatment

### BACKGROUND

Clinical studies and practice have shown that reducing pressure in proximity to a tissue site can augment and accelerate growth of new tissue at the tissue site. The applications of this phenomenon are numerous, but it has proven particularly advantageous for treating wounds. Regardless of the etiology of a wound, whether trauma, surgery, or another cause, proper care of the wound is important to the outcome. Treatment of wounds or other tissue with reduced pressure may be commonly referred to as "negative -pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, and micro-deformation of tissue at a wound site. Together, these benefits can increase development of granulation tissue and reduce healing times.

There is also widespread acceptance that cleansing a tissue site can be highly beneficial for new tissue growth. For example, a wound or a cavity can be washed out with a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy and/or instillation therapy are widely known, improvements to therapy systems, components, and processes may benefit healthcare providers and patients.
US2020253788 discloses a negative pressure wound treatment (NPWT) dressing system for shoulder incisions, including a wound dressing, an immobilization device, and a negative pressure source. The wound dressing features drape and manifold layers.

### BRIEF SUMMARY

The invention is defined by the independent claim. A selection of optional features is set out in the dependent claims.

### BRIEF DESCRIPTION OF THE DRAWINGS

Figure 1 is a block diagram of an example embodiment of a therapy system including an example dressing that can provide negative-pressure treatment and instillation treatment in accordance with this specification;
Figure 2 is an exploded view of an example pressure offloading component which may be used with the therapy system of Figure 1;
Figure 3 is an isometric view of an assembled example of the pressure offloading component of Figure 2;
Figure 4 is a cross-sectional view of an example pressure offloading component of Figure 3, taken at line 4-4, illustrating additional details associated with some embodiments;
Figure 5 is a cross-sectional view, illustrating additional details that may be associated with some embodiments of the pressure offloading component shown in Figure 4;
Figure 6 is an exploded view of an example embodiment of the pressure offloading component of Figure 2 which further includes an additional thermoformed layer;
Figure 7 is an isometric cutaway view of an assembled example of the pressure offloading component of Figure 6 with a portion of the embossed layer removed to show a portion of the embossed layer;
Figure 8 is a cross-sectional view of an example pressure offloading component of Figure 7, taken at line 8-8, illustrating additional details associated with some embodiments;
Figure 9 is a cross-sectional view, illustrating additional details that may be associated with some embodiments of the pressure offloading component shown in Figure 4;
Figure 10 is an exploded view of an example embodiment of a therapy system of Figure 1 including a pressure offloading component in accordance with this specification; and
Figure 11 is an isometric view of an assembled example of the therapy system of Figure 10.

### DESCRIPTION OF EXAMPLE EMBODIMENTS

The following description of example embodiments provides information that enables a person skilled in the art to make and use the subject matter set forth in the appended claims, but it may omit certain details already well-known in the art. The following detailed description is, therefore, to be taken as illustrative and not limiting.

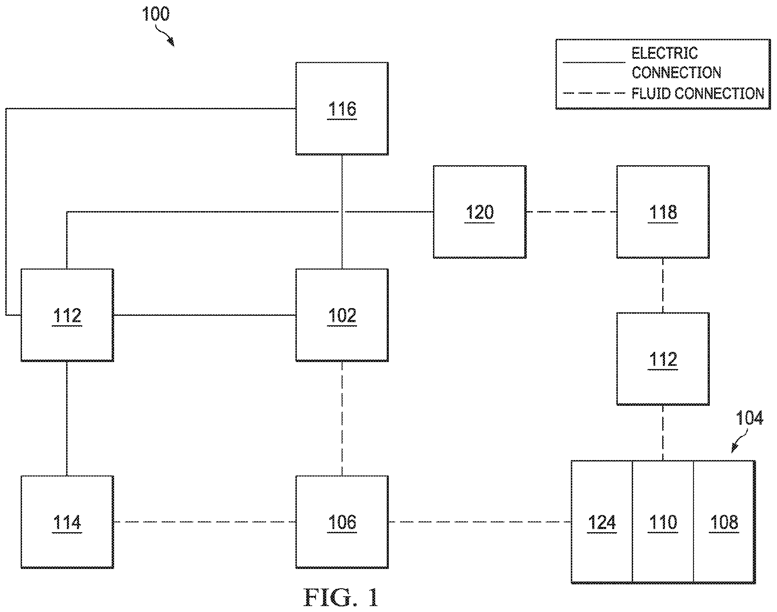

Figure 1 is a block diagram of an example embodiment of a therapy system 100 that can provide negative-pressure therapy and instillation treatment in accordance with this specification.

The term "tissue site" in this context broadly refers to a wound, defect, or other treatment target located on or within tissue, including, but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness bums, ulcers (such as diabetic, pressure, or venous insufficiency ulcers), flaps, and grafts, for example. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted.

The therapy system 100 may include a source or supply of negative pressure, such as a negative-pressure source 102, and one or more distribution components. A distribution component is preferably detachable and may be disposable, reusable, or recyclable. A dressing, such as a dressing 104, and a fluid container, such as a container 106, are examples of distribution components that may be associated with some examples of the therapy system 100. As illustrated in the example of Figure 1, the dressing 104 may include a tissue interface 108, a cover 110, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include interfaces or fluid ports to facilitate coupling and de-coupling other components. In some embodiments, for example, a dressing interface may facilitate coupling a fluid conductor to the dressing 104. For example, such a dressing interface may be a SENSAT.R.A.C.^{™} Pad available from Kinetic Concepts, Inc. of San Antonio, Texas.

The therapy system 100 may also include a regulator or controller, such as a controller 112. Additionally, the therapy system 100 may include sensors to measure operating parameters and provide feedback signals to the controller 112 indicative of the operating parameters. As illustrated in Figure 1, for example, the therapy system 100 may include a first sensor 114 and a second sensor 116 coupled to the controller 112.

The therapy system 100 may also include a source of instillation solution. For example, a solution source 118 may be fluidly coupled to the dressing 104, as illustrated in the example embodiment of Figure 1. The solution source 118 may be fluidly coupled to a positive-pressure source such as a positive-pressure source 120, a negative-pressure source such as the negative-pressure source 102, or both in some embodiments. A regulator, such as an instillation regulator, may also be fluidly coupled to the solution source 118 and the dressing 104 to ensure proper dosage of instillation solution (e.g. saline) to a tissue site. For example, the instillation regulator 122 may include a piston that can be pneumatically actuated by the negative-pressure source 102 to draw instillation solution from the solution source during a negative-pressure interval and to instill the solution to a dressing during a venting interval. Additionally or alternatively, the controller 112 may be coupled to the negative-pressure source 102, the positive-pressure source 120, or both, to control dosage of instillation solution to a tissue site. In some embodiments, the instillation regulator 122 may also be fluidly coupled to the negative-pressure source 102 through the dressing 104, as illustrated in the example of Figure 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 102 may be combined with the controller 112, the solution source 118, and other components into a therapy unit.

In general, components of the therapy system 100 may be coupled directly or indirectly. For example, the negative-pressure source 102 may be directly coupled to the container 106 and may be indirectly coupled to the dressing 104 through the container 106. Coupling may include fluid, mechanical, thermal, electrical, or chemical coupling (such as a chemical bond), or some combination of coupling in some contexts. For example, the negative-pressure source 102 may be electrically coupled to the controller 112 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 102, may be a reservoir of air at a negative pressure or may be a manual or electrically-powered device, such as a vacuum pump, a suction pump, a wall suction port available at many healthcare facilities, or a micro-pump, for example. "Negative pressure" generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 102 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between - 50 mm Hg (-6.7 kPa) and -300 mm Hg (-39.9 kPa).

The container 106 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 112, may be a microprocessor or computer programmed to operate one or more components of the therapy system 100, such as the negative-pressure source 102. In some embodiments, for example, the controller 112 may be a microcontroller, which generally includes an integrated circuit containing a processor core and a memory programmed to directly or indirectly control one or more operating parameters of the therapy system 100. Operating parameters may include the power applied to the negative-pressure source 102, the pressure generated by the negative-pressure source 102, or the pressure distributed to the tissue interface 108, for example. The controller 112 is also preferably configured to receive one or more input signals, such as a feedback signal, and programmed to modify one or more operating parameters based on the input signals.

Sensors, such as the first sensor 114 and the second sensor 116, are generally known in the art as any apparatus operable to detect or measure a physical phenomenon or property, and generally provide a signal indicative of the phenomenon or property that is detected or measured. For example, the first sensor 114 and the second sensor 116 may be configured to measure one or more operating parameters of the therapy system 100. In some embodiments, the first sensor 114 may be a transducer configured to measure pressure in a pneumatic pathway and convert the measurement to a signal indicative of the pressure measured. In some embodiments, for example, the first sensor 114 may be a piezo-resistive strain gauge. The second sensor 116 may optionally measure operating parameters of the negative-pressure source 102, such as a voltage or current, in some embodiments. Preferably, the signals from the first sensor 114 and the second sensor 116 are suitable as an input signal to the controller 112, but some signal conditioning may be appropriate in some embodiments. For example, the signal may need to be filtered or amplified before it can be processed by the controller 112. Typically, the signal is an electrical signal, but may be represented in other forms, such as an optical signal.

The tissue interface 108 can be generally adapted to partially or fully contact a tissue site. The tissue interface 108 may take many forms, and may have many sizes, shapes, or thicknesses, depending on a variety of factors, such as the type of treatment being implemented or the nature and size of a tissue site. For example, the size and shape of the tissue interface 108 may be adapted to the contours of deep and irregular shaped tissue sites. Any or all of the surfaces of the tissue interface 108 may have an uneven, coarse, or jagged profile.

In some embodiments, the tissue interface 108 may include or may be a manifold as further described herein. A manifold in this context may include a means for communicating fluid relative to a tissue site under pressure, such as a means for collecting fluid from or distributing fluid to the tissue site. For example, a manifold may be adapted to receive negative pressure from a source and distribute negative pressure through multiple apertures across the tissue interface 108, which may have the effect of collecting fluid from a tissue site and drawing the fluid toward the source. In some embodiments, the fluid path may be reversed or a secondary fluid path may be provided to facilitate delivering fluid, such as fluid from a source of instillation solution, across a tissue site.

In some illustrative embodiments, a manifold may include a plurality of pathways, which can be interconnected to improve distribution or collection of fluids. In some illustrative embodiments, a manifold may include a porous material having interconnected fluid pathways. Examples of suitable porous material that can be adapted to form interconnected fluid pathways (e.g., channels) may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, a manifold may additionally or alternatively include projections that form interconnected fluid pathways. For example, a manifold may be molded to provide surface projections that define interconnected fluid pathways.

In some embodiments, the tissue interface 108 may include reticulated foam having pore sizes and free volume that may vary according to needs of a prescribed therapy. For example, reticulated foam having a free volume of at least 90% may be suitable for many therapy applications, and foam having an average pore size in a range of 400-600 microns (40-50 pores per inch) may be particularly suitable for some types of therapy. The tensile strength of the tissue interface 108 may also vary according to needs of a prescribed therapy. For example, the tensile strength of foam may be increased for instillation of topical treatment solutions. The 25% compression load deflection of the tissue interface 108 may be at least 0.35 pounds per square inch, (1 PSI = 6,895 Pascals) and the 65% compression load deflection may be at least 0.43 pounds per square inch. In some embodiments, the tensile strength of the tissue interface 108 may be at least 10 pounds per square inch. The tissue interface 108 may have a tear strength of at least 2.5 pounds per inch. In some embodiments, the tissue interface may be foam including of polyols such as polyester or polyether, isocyanate such as toluene diisocyanate, and polymerization modifiers such as amines and tin compounds. In some examples, the tissue interface 108 may be reticulated polyurethane foam such as found in GRANUFOAM^{™} dressing or V.A.C. VERAFLO^{™} dressing, both available from Kinetic Concepts, Inc. of San Antonio, Texas.

The thickness of the tissue interface 108 may also vary according to needs of a prescribed therapy. For example, the thickness of the tissue interface may be decreased to reduce tension on peripheral tissue. The thickness of the tissue interface 108 can also affect the conformability of the tissue interface 108. In some embodiments, a thickness in a range of about 5 millimeters to 10 millimeters may be suitable.

The tissue interface 108 may be either hydrophobic or hydrophilic. In an example in which the tissue interface 108 may be hydrophilic, the tissue interface 108 may also wick fluid away from a tissue site, while continuing to distribute negative pressure to the tissue site. The wicking properties of the tissue interface 108 may draw fluid away from a tissue site by capillary flow or other wicking mechanisms. An example of a hydrophilic material that may be suitable is a polyvinyl alcohol, open-cell foam such as V.A.C. WHITEFOAM^{™} dressing available from Kinetic Concepts, Inc. of San Antonio, Texas. Other hydrophilic foams may include those made from polyether. Other foams that may exhibit hydrophilic characteristics include hydrophobic foams that have been treated or coated to provide hydrophilicity.

In some embodiments, the tissue interface 108 may be constructed from bioresorbable materials. Suitable bioresorbable materials may include, without limitation, a polymeric blend of polylactic acid (PLA) and polyglycolic acid (PGA). The polymeric blend may also include, without limitation, polycarbonates, polyfumarates, and capralactones. The tissue interface 108 may further serve as a scaffold for new cell-growth, or a scaffold material may be used in conjunction with the tissue interface 108 to promote cell-growth. A scaffold is generally a substance or structure used to enhance or promote the growth of cells or formation of tissue, such as a three-dimensional porous structure that provides a template for cell growth. Illustrative examples of scaffold materials include calcium phosphate, collagen, PLA/PGA, coral hydroxy apatites, carbonates, or processed allograft materials.

In some embodiments, the cover 110 may provide a bacterial barrier and protection from physical trauma. The cover 110 may also be constructed from a material that can reduce evaporative losses and provide a fluid seal between two components or two environments, such as between a therapeutic environment and a local external environment. The cover 110 may include, for example, an elastomeric film or membrane that can provide a seal adequate to maintain a negative pressure at a tissue site for a given negative-pressure source. The cover 110 may have a high moisture-vapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, an MVTR up to 5,000 grams per square meter per twenty-four hours may provide effective breathability and mechanical properties.

In some example embodiments, the cover 110 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 110 may include, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polymide copolymers. Such materials are commercially available as, for example, Tegaderm^{®} drape, commercially available from 3M Company, Minneapolis Minnesota; polyurethane (PU) drape, commercially available from Avery Dennison Corporation, Pasadena, California; polyether block polyamide copolymer (PEBAX), for example, from Arkema S.A., Colombes, France; and Inspire 2301 and Inspire 2327 polyurethane films, commercially available from Expopack Advanced Coatings, Wrexham, United Kingdom. In some embodiments, the cover 110 may include INSPIRE 2301 having an MVTR (upright cup technique) of 2600 g/m²/24 hours and a thickness of about 30 microns.

An attachment device may be used to attach the cover 110 to an attachment surface, such as undamaged epidermis, a gasket, or another cover. The attachment device may take many forms. For example, an attachment device may be a medically-acceptable, pressure-sensitive adhesive configured to bond the cover 110 to epidermis around a tissue site. In some embodiments, for example, some or all of the cover 110 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 118 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfur-based solutions, biguanides, cationic solutions, and isotonic solutions.

In operation, the tissue interface 108 may be placed within, over, on, or otherwise proximate to a tissue site. If the tissue site is a wound, for example, the tissue interface 108 may partially or completely fill the wound, or it may be placed over the wound. The cover 110 may be placed over the tissue interface 108 and sealed to an attachment surface near a tissue site. For example, the cover 110 may be sealed to undamaged epidermis peripheral to a tissue site. Thus, the dressing 104 can provide a sealed therapeutic environment proximate to a tissue site, substantially isolated from the external environment, and the negative-pressure source 102 can reduce pressure in the sealed therapeutic environment.

In general, exudate and other fluid flow toward lower pressure along a fluid path. Thus, the term "downstream" typically implies a position in a fluid path relatively closer to a source of negative pressure or further away from a source of positive pressure. Conversely, the term "upstream" implies a position relatively further away from a source of negative pressure or closer to a source of positive pressure. Similarly, it may be convenient to describe certain features in terms of fluid "inlet" or "outlet" in such a frame of reference. This orientation is generally presumed, without limitation, for purposes of describing various features and components herein. However, the fluid path may also be reversed in some applications, such as by substituting a positive-pressure source for a negative-pressure source.

Negative pressure applied to the tissue site through the tissue interface 108 in the sealed therapeutic environment can induce macro-strain and micro-strain in the tissue site. Negative pressure can also remove exudate and other fluid from a tissue site, which can be collected in container 106.

In some embodiments, the controller 112 may receive and process data from one or more sensors, such as the first sensor 114. The controller 112 may also control the operation of one or more components of the therapy system 100 to manage the pressure delivered to the tissue interface 108. In some embodiments, controller 112 may include an input for receiving a desired target pressure and may be programmed for processing data relating to the setting and inputting of the target pressure to be applied to the tissue interface 108. In some example embodiments, the target pressure may be a fixed pressure value set by an operator as the target negative pressure desired for therapy at a tissue site and then provided as input to the controller 112. The target pressure may vary from tissue site to tissue site based on the type of tissue forming a tissue site, the type of injury or wound (if any), the medical condition of the patient, and the preference of the attending physician. After selecting a desired target pressure, the controller 112 can operate the negative-pressure source 102 in one or more control modes based on the target pressure and may receive feedback from one or more sensors to maintain the target pressure at the tissue interface 108.

In some embodiments, the controller 112 may have a continuous pressure mode, in which the negative-pressure source 102 is operated to provide a constant target negative pressure for the duration of treatment or until manually deactivated. Additionally or alternatively, the controller may have an intermittent pressure mode. In example embodiments, the controller 112 can operate the negative-pressure source 102 to cycle between a target pressure and atmospheric pressure. For example, the target pressure may be set at a value of 135 mmHg for a specified period of time (e.g., 5 min), followed by a specified period of time (e.g., 2 min) of deactivation. The cycle can be repeated by activating the negative-pressure source 102, which can form a square wave pattern between the target pressure and atmospheric pressure.

In some example embodiments, the increase in negative-pressure from ambient pressure to the target pressure may not be instantaneous. For example, the negative-pressure source 102 and the dressing 104 may have an initial rise time. The initial rise time may vary depending on the type of dressing and therapy equipment being used. For example, the initial rise time for one therapy system may be in a range of about 20-30 mmHg/second and in a range of about 5-10 mmHg/second for another therapy system. If the therapy system 100 is operating in an intermittent mode, the repeating rise time may be a value substantially equal to the initial rise time.

In some example dynamic pressure control modes, the target pressure can vary with time. For example, the target pressure may vary in the form of a triangular waveform, varying between a negative pressure of 50 and 135 mmHg with a rise rate of negative pressure set at a rate of 25 mmHg/min. and a descent rate set at 25 mmHg/min. In other embodiments of the therapy system 100, the triangular waveform may vary between negative pressure of 25 and 135 mmHg with a rise rate of about 30 mmHg/min. and a descent rate set at about 30 mmHg/min.

In some embodiments, the controller 112 may control or determine a variable target pressure in a dynamic pressure mode, and the variable target pressure may vary between a maximum and minimum pressure value that may be set as an input prescribed by an operator as the range of desired negative pressure. The variable target pressure may also be processed and controlled by the controller 112, which can vary the target pressure according to a predetermined waveform, such as a triangular waveform, a sine waveform, or a saw-tooth waveform. In some embodiments, the waveform may be set by an operator as the predetermined or time-varying negative pressure desired for therapy.

In some embodiments, the controller 112 may receive and process data, such as data related to instillation solution provided to the tissue interface 108. Such data may include the type of instillation solution prescribed by a clinician, the volume of fluid or solution to be instilled to a tissue site ("fill volume"), and the amount of time prescribed for leaving solution at a tissue site ("dwell time") before applying a negative pressure to the tissue site. The fill volume may be, for example, between 10 and 500 mL, and the dwell time may be between one second to 30 minutes. The controller 112 may also control the operation of one or more components of the therapy system 100 to instill solution. For example, the controller 112 may manage fluid distributed from the solution source 118 to the tissue interface 108. In some embodiments, fluid may be instilled to a tissue site by applying a negative pressure from the negative-pressure source 102 to reduce the pressure at the tissue site, drawing solution into the tissue interface 108. In some embodiments, solution may be instilled to a tissue site by applying a positive pressure from the positive-pressure source 120 to move solution from the solution source 118 to the tissue interface 108. Additionally or alternatively, the solution source 118 may be elevated to a height sufficient to allow gravity to move solution into the tissue interface 108.

The controller 112 may also control the fluid dynamics of instillation by providing a continuous flow of solution or an intermittent flow of solution. Negative pressure may be applied to provide either continuous flow or intermittent flow of solution. The application of negative pressure may be implemented to provide a continuous pressure mode of operation to achieve a continuous flow rate of instillation solution through the tissue interface 108, or it may be implemented to provide a dynamic pressure mode of operation to vary the flow rate of instillation solution through the tissue interface 108. Alternatively, the application of negative pressure may be implemented to provide an intermittent mode of operation to allow instillation solution to dwell at the tissue interface 108. In an intermittent mode, a specific fill volume and dwell time may be provided depending, for example, on the type of tissue site being treated and the type of dressing being utilized. After or during instillation of solution, negative-pressure treatment may be applied. The controller 112 may be utilized to select a mode of operation and the duration of the negative pressure treatment before commencing another instillation cycle by instilling more solution.

Some therapy systems may include structures that can interfere with patient comfort and safety. For example, provision of therapy to an area of the patient where force is regularly applied, for example, the dorsal surface of a patient in a supine position, may require special consideration for comfortable and safe application of an appropriate therapy. For some therapy systems, it may be beneficial to increase the surface area of a dressing interface and/or a fluid conductor coupled to the dressing in a plane normal to the force applied to the patient. By increasing the surface area of the dressing interface and/or the fluid conductor, the pressure applied to the patient across an area where the dressing interface and/or the fluid conductor is in direct or indirect contact with the patient may be reduced. However, conventional systems that increase the surface area of a dressing interface are often prohibitively expensive and compromise performance.

These issues and others may be solved by the therapy system 100 which provides a pressure-offloading component 124. In some examples, the pressure-offloading component 124 may have a height greater than a height of the dressing interface and/or the fluid conductor in a direction parallel with the force applied to the patient, and a surface area greater than the surface area of the dressing interface and/or the fluid conductor in the plane normal to the force applied to the patient. The height of the pressure-offloading component 124 may permit the dressing interface and/or fluid conductor to avoid contact with the surface providing the force to the patient. Instead, the force may be provided to the surface area of the pressure-offloading component 124 in the plane normal to the force. The surface area of the pressure-offloading component 124 reduces the pressure experienced by the patient at any point in direct or indirect contact with the pressure-offloading component 124.

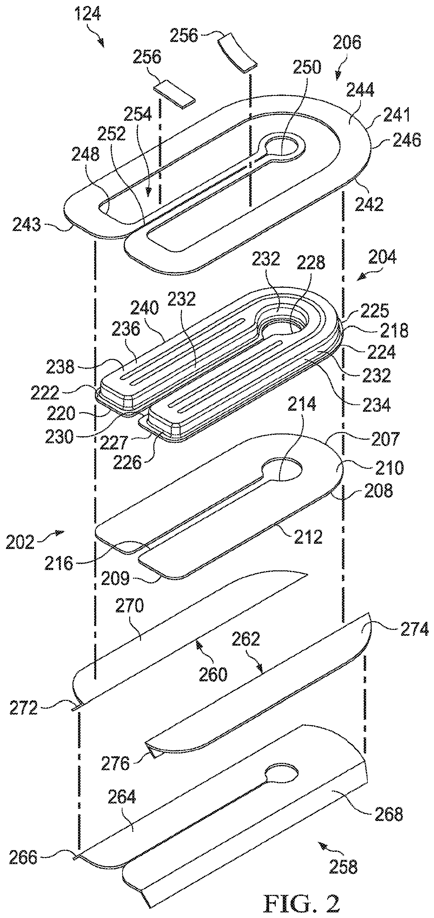

Figure 2 is an exploded view of an example pressure-offloading component 124 which may be used with the therapy system 100 of Figure 1. The pressure-offloading component 124 may include a sealing layer, such as film layer 202, a thermoformed layer, such as embossed layer 204, and a cover layer, such as film layer 206. The film layer 202 may be substantially sheet-like having a first end 207 and a second end 209 opposite the first end 207. In some embodiments, the film layer 202 may be substantially rectangular having the first end 207 and the second end 209 comprise a width of the film layer 202 and the first end 207 and the second end 209 being separated by a length of the film layer 202. In some embodiments, the first end 207 may be semi-circular or curved. The second end 209 may be angular or squared-off. In other embodiments, the first end 207 and the second end 209 may have a same shape. The first end 207 and the second end 209 may have other shapes such as ovular, square, or amorphous.

In some embodiments, the film layer 202 can include a first surface 208 and a second surface 210 opposite the first surface 208. In some embodiments, the first surface 208 may be configured to be oriented to face a tissue site, and the second surface 210 may be configured to be oriented to face away from the tissue site. The first surface 208 and the second surface 210 may be substantially planar surfaces. In some examples, the first surface 208 and the second surface 210 may be substantially parallel. In some examples, the film layer 202 may have a uniform thickness between the first surface 208 and the second surface 210. In some examples, the film layer 202 may have a thickness in a range of between about 25 micrometers to about 50 micrometers.

The film layer 202 may include an edge or perimeter, such as a periphery 212. For example, the periphery 212 may be defined by an outer perimeter of the first surface 208 or the second surface 210. In some examples, the periphery 212 may define a substantially rectangular shape having a first, semi-circular or curved end and a second, angular or squared-off end. In some examples, the first end of the periphery 212 may be separated from the second end of the periphery 212 by a length. In other examples, the first end of the periphery 212 and the second end of the periphery 212 may have a same shape. The periphery 212 may define other shapes, such as an ovular, a square, or an amorphous shape.

In some examples, an opening, such as aperture 214, may be formed through the film layer 202 near the first end 207. In some examples, the periphery 212 may define the aperture 214. In some examples, the aperture 214 may be circular. In other embodiments, the aperture 214 have other shapes, such as elliptical, square, triangular, or amorphous shapes. In some examples, as shown in Figure 2, the aperture 214 may be concentric with the first end 207. For example, the film layer 202 may have a uniform width between the aperture 214 and the periphery 212 at the first end 207. In other embodiments, the aperture 214 may not be concentric with the first end 207 or the aperture 214 and the first end 207 may be differently shaped, causing a width between the aperture 214 and the periphery 212 at the first end 207 to be variable.

The film layer 202 may also include a cutaway portion, such as notch 216. In some examples, the periphery 212 may define the notch 216. In some examples, as shown in Figure 2, the notch 216 may extend from the second end 209 of the film layer 202 to the aperture 214. In some examples, the notch 216 may be a slot, extending into the film layer 202 from the second end 209 parallel to the length of the film layer 202 between the first end 207 and the second end 209. In some examples, a portion of the periphery 212 may define the aperture 214 and the notch 216.

The film layer 202 may be formed from a material capable of providing a fluid seal between two components and/or two environments. In some examples, the film layer 202 may be formed from any of the materials previously described with respect to cover 110. In some examples, the film layer 202 may be a flexible material capable of providing a fluid seal, such as polymer drape. In some examples, the film layer 202 may include one or more of the following materials: polyurethane; cellulosics; hydrophilic polyamides; polyvinyl alcohol; polyvinyl pyrrolidone; hydrophilic acrylics; silicones; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate; co-polyester; and/or polyether block polymide copolymers.

In some examples, the film layer 202 may include an attachment device, such as an adhesive. The attachment device can be disposed on some or all of the first surface 208. In some examples, the attachment device of the film layer 202 may be any attachment device previously described with respect to the attachment device of the cover 110. For example, the attachment device may be a medically-acceptable, pressure sensitive adhesive, an acrylic adhesive, such as an acrylic adhesive with a coating weight of about 25-65 g.s.m., a double-sided tape, a paste, a hydrocolloid, a hydrogel, a silicone gel, or an organogel.

The embossed layer 204 may be a substantially sheet-like structure with three-dimensional features suitable for forming a cavity. When assembled, the embossed layer 204 and the film layer 202 may define a cell. The embossed layer 204 may have a first end 225 and a second end 227 opposite the first end 225. In some examples, outer periphery 224the embossed layer 204 may generally define a substantially rectangular shape with the first end 225 and the second end 227 being separated by a length of the embossed layer 204. In some examples, the first end 225 may be substantially semi-circular or substantially curved. In some examples, the second end 227 may be substantially angular or squared-off. In some examples, the first end 225 and the second end 227 may have substantially the same shape. In some examples, the first end 225 and/or the second end 227 may have other shapes, such as ovular, squared, or amorphous shapes.

The embossed layer 204 may also include a planar portion 218. The planar portion 218 may include a first surface 220 opposite a second surface 222. In some examples, the first surface 220 may be configured to be oriented to face the tissue site, and the second surface 222 may be configured to face away from the tissue site. The first surface 220 and the second surface 222 may be substantially planar surfaces. In some examples, the first surface 220 and the second surface 222 may be substantially parallel. In some examples, the planar portion 218 may have a substantially uniform thickness between the first surface 220 and the second surface 222. In some examples, the planar portion 218 may have a uniform thickness in a range of between about 250 micrometers to about 3,000 micrometers. For example, the planar portion 218 may have a uniform thickness in a range of about 500 micrometers to about 1,000 micrometers. In some examples, the planar portion 218 may have a uniform thickness in a range of about 800 micrometers to about 3,000 micrometers. In some examples, the planar portion 218 may have a uniform thickness in a range of about 250 micrometers and about 1,000 micrometers. In some examples, the planar portion 218 may have a uniform thickness of about 500 micrometers. The planar portion 218 may have an outer edge or perimeter, such as an outer periphery 224. The outer periphery 224 may define an outline or a shape of the planar portion 218.

The embossed layer 204 may include an opening, such as aperture 228, near the first end 225. In some examples, the outer periphery 224 of the planar portion 218 may define the aperture 228. In some examples, the aperture 228 may be circular. In some examples, the aperture 228 may have other shapes, such as elliptical, triangular, rectangular, polyhedral, or amorphous shapes. In some examples, as shown in Figure 2, the aperture 228 may be concentric with the first end 225. For example, the embossed layer 204 may have a uniform width between the aperture 228 and the portion of the outer periphery 224 at the first end 225. In some examples, the aperture 228 may not be concentric with the first end 225. In some examples, the aperture 228 may not have a similar shape to the shape of the first end 225. In some examples, the width between the aperture 228 and the portion of the outer periphery 224 at the first end 225 may be variable.

The embossed layer 204 may also include a cutaway portion, such as a notch 230. In some examples, the outer periphery 224 of the planar portion 218 may define the notch 230. In some examples, as shown in Figure 2, the notch 230 may extend from the second end 227 to the aperture 228. In some examples, the notch 230 may be a slot, extending into the embossed layer 204 from the second end 227 parallel to the length of the embossed layer 204. In some examples, a portion of the outer periphery 224 may define the aperture 228 and the notch 230.

The planar portion 218 may have an inner edge or perimeter, such as an inner periphery 226. In some examples, the planar portion 218 may be defined as the material between the first surface 220, the second surface 222, the outer periphery 224, and the inner periphery 226. In some examples, the inner periphery 226 may be substantially geometrically similar to the outer periphery 224, and scaled to contain a smaller area. In some examples, the inner periphery 226 may be substantially parallel to the outer periphery 224. In some examples, a distance between the inner periphery 226 and the outer periphery 224 may remain constant. In some examples, the inner periphery 226 and the outer periphery 224 may define dissimilar or different shapes.

The embossed layer 204 may further include a wall or a walled portion 232. The walled portion 232 may be a substantially sheet-like structure including a first surface (not shown) opposite a second surface 234. In some examples, the first surface of the walled portion 232 may be configured to be oriented to face substantially towards the cavity defined by the embossed layer 204, and the second surface 234 of the walled portion 232 may be configured to be oriented to face substantially away from the cavity defined by the embossed layer 204. In some examples, the first surface of the walled portion 232 may be substantially planar. In some examples, the first surface of the walled portion 232 may include substantially flat planar portions and substantially curved planar portions. In some examples, the second surface 234 may be substantially planar. In some examples, the second surface 234 may include substantially flat planar portions and substantially curved planar portions. In some examples, the first surface of the walled portion 232 and the second surface 234 of the walled portion 232 may be substantially parallel. In some examples, the walled portion 232 may have a uniform thickness in a range of between about 250 micrometers to about 3,000 micrometers. For example, the walled portion 232 may have a uniform thickness in a range of about 500 micrometers to about 1,000 micrometers. In some examples, the walled portion 232 may have a uniform thickness in a range of about 800 micrometers to about 3,000 micrometers. In some examples, the walled portion 232 may have a uniform thickness in a range of about 250 micrometers and about 1,000 micrometers. In some examples, the walled portion 232 may have a uniform thickness of about 500 micrometers.

The walled portion 232 may include a base at an edge of the first surface and/or the second surface 234 proximate to the second surface 222 of the planar portion 218. In some examples, the walled portion 232 may include a top at the edge of the first surface and/or the second surface 234 distal from the second surface 222 of the planar portion 218. In some examples, the walled portion 232 may be joined to the planar portion 218 at the base. The base of the walled portion 232 may follow a path defined by the inner periphery 226 of the planar portion 218. In some examples, the plane defined by the first surface of the walled portion 232 may extend in a direction different from the plane defined by the second surface 222 of the planar portion 218. For example, the plane defined by the first surface of the walled portion 232 may be substantially orthogonal to the plane defined by the second surface 222 of the planar portion 218 where the walled portion 232 is joined to the planar portion 218. In some examples, the plane defined by the second surface 234 of the walled portion 232 may extend in a direction different from the plane defined by the second surface 222 of the planar portion 218. For example, the plane defined by the second surface 234 of the walled portion 232 may be substantially orthogonal to the plane defined by the second surface 222 of the planar portion 218 where the walled portion 232 is joined to the planar portion 218.

The embossed layer 204 may further include a second planar portion, such as planar portion 236. The planar portion 236 may be a substantially sheet-like structure, including a first surface (not shown) opposite a second surface 238. The first surface of the planar portion 236 may be configured to be oriented to face the tissue site, and the second surface 238 of the planar portion 236 may be configured to be oriented to face away from the tissue site. In some examples, the first surface and the second surface 238 of the planar portion 236 may be substantially planar surfaces. In some examples, the first surface of the planar portion 236 may be parallel to the second surface 238 of the planar portion 236. In some examples, the planar portion 236 may have a uniform thickness between the first surface and the second surface 238. In some examples, the planar portion 236 may have a uniform thickness in a range of between about 250 micrometers to about 3,000 micrometers. For example, the planar portion 236 may have a uniform thickness in a range of about 500 micrometers to about 1,000 micrometers. In some examples, the planar portion 236 may have a uniform thickness in a range of about 800 micrometers to about 3,000 micrometers. In some examples, the planar portion 236 may have a uniform thickness in a range of about 250 micrometers and about 1,000 micrometers. In some examples, the planar portion 236 may have a uniform thickness of about 500 micrometers.

The planar portion 236 may include an edge or perimeter, such as a periphery 240. In some examples, the periphery 240 may be defined by an outer perimeter of the first surface or the second surface 238 of the planar portion 236. In some examples, the periphery 240 of the planar portion 236 may be joined to the walled portion 232 at the top of the walled portion 232. In some examples, the plane defined by the first surface of the planar portion 236 may be substantially parallel to the plane defined by the second surface 222 of the planar portion 218. In some examples, the plane defined by the second surface 238 of the planar portion 236 may be substantially parallel to the plane defined by the second surface 222 of the planar portion 218. In some examples, the embossed layer 204 may define a cavity or a partially enclosed space between the first surface of the walled portion 232 and the first surface of the planar portion 236.

In some examples, the outer periphery 224 of the planar portion 218 may be substantially geometrically similar to the periphery 212 of the film layer 202. In some examples, the outer periphery 224 of the planar portion 218 may be substantially parallel to the periphery 212 of the film layer 202. In some examples, the outer periphery 224 may be substantially coextensive with the periphery 212 of the film layer 202.

In some examples, a cross-section of the first surface and/or the second surface 234 of the walled portion 232 taken in a plane parallel to the second surface 222 of the planar portion 218 may be substantially geometrically similar to the path defined by the inner periphery 226 of the planar portion 218. In some examples, a cross-section of the first surface and/or the second surface 234 of the walled portion 232 taken in a plane parallel to the second surface 222 of the planar portion 218 may be substantially parallel to the path defined by the inner periphery 226 of the planar portion 218. In some examples, a cross-section of the second surface 234 of the walled portion 232 taken in a plane parallel to the second surface 222 of the planar portion 218 may be substantially coextensive with the path defined by the inner periphery 226 of the planar portion 218. In some examples, the cross-section of the first surface/and or the second surface 234 of the walled portion 232 taken in a plane parallel to the second surface 222 of the planar portion 218 may define an aperture and a notch substantially geometrically similar to the aperture 228 and the notch 230 formed through the planar portion 218. In some examples, the cross-section of the first surface/and or the second surface 234 of the walled portion 232 taken in a plane parallel to the second surface 222 of the planar portion 218 may define an aperture and a notch substantially parallel to the aperture 228 and the notch 230 formed through the planar portion 218.

In some examples, the embossed layer 204 may include an attachment device, such as an adhesive. The attachment device may be disposed on some or all of the first surface 220 of the planar portion 218. In some examples, the attachment device of the embossed layer 204 may be any attachment device previously described with respect to the attachment devices of the cover 110 and/or the film layer 202. For example, the attachment device may be a medically-acceptable, pressure sensitive adhesive, an acrylic adhesive, such as an acrylic adhesive with a coating weight of about 25-65 g.s.m., a double-sided tape, a paste, a hydrocolloid, a hydrogel, a silicone gel, or an organogel.

In some examples, the embossed layer 204 may be formed from any of the materials previously described with respect to cover 110. In some examples, the embossed layer 204 may be formed from a non-porous polymeric film which may include any flexible material that can be manipulated to form a defined shape which may enclose a space, including thermoplastics. Non-limiting examples of suitable thermoplastics include polyethylene homopolymers (such as low-density polyethylene (LDPE) or high-density polyethylene (HDPE)) and/or polyethylene copolymers (such as ionomers, EVA, EMA, heterogeneous (Zeigler-Natta catalyzed) ethylene/alpha-olefin copolymers, and homogenous (metallocene, single-site catalyzed) ethylene/alpha olefin copolymers). Ethylene/alphaolefin copolymers are copolymers are copolymers of ethylene with one or more comonomers selected from C₃ to C₂₀ alpha-olefins, such as 1-butene, 1-pentene, 1-hexene, 1-octene, methyl pentene in which the polymer molecules include long chains with relatively few side chain branches. Examples of suitable ethyle/alpha-olefin copolymers include linear low-density polyethylene (LLDPE), linear medium-density polyethylene (LMDPE), very-low-density polyethylene (VLDPE), and ultra-low-density polyethylene (ULDPE). In some examples, the embossed layer 204 may be formed from or include polypropylene homopolymers, polypropylene copolymers, polyesters, polystyrenes, polyamides, and polycarbonates. In some examples, the embossed layer 204 may be formed from an HDPE material with an ultimate tensile strength of about 37 MPa and a yield strength in a range of about 26-33 MPa. In some examples, the embossed layer 204 may be formed from a thermoplastic polyurethane film, such PLATILON^{®} thermoplastic films available from Convestro AG. For example, a thermoplastic polyurethane film having an ultimate tensile strength of about 60 MPa and a yield strength of greater than about 10 MPa may be used.

The film layer 206 may be substantially sheet-like having a first end 241 opposite a second end 243. In some examples, the film layer 206 may be substantially rectangular having the first end 241 and the second end 243 define a width of the film layer 206. In some examples, the first end 241 and the second end 243 may be separated by a length of the film layer 206. In some examples, the first end 241 may be semi-circular or curved. The second end 243 may be angular or squared-off. In other embodiments, the first end 241 and the second end 243 may have a same shape. The first end 241 and the second end 243 may have other shapes, such as ovular, square, or amorphous.

In some examples, the film layer 206 can include a first surface 242 opposite a second surface 244. In some examples, the first surface 242 may be configured to be oriented to face a tissue site, and the second surface 244 may be configured to be oriented to face away from a tissue site. The first surface 242 and the second surface 244 may be substantially planar surfaces. In some examples, the first surface 242 and the second surface 244 may be substantially parallel. In some examples, the film layer 206 may have a uniform thickness between the first surface 242 and the second surface 244. In some examples, the film layer 206 may have a thickness in a range of between about 25 micrometers to about 50 micrometers.

The film layer 206 may include an outer edge or perimeter, such as periphery 246. For example, the periphery 246 may be defined by an outer perimeter of the first surface 242 or the second surface 244. In some examples, the periphery 246 may define a substantially rectangular shape having a first, semi-circular or curved end and a second, angular or squared-off end. In some examples, the first end of the periphery 246 may be separated from the second end of the periphery 246 by a length. In other examples, the first end of the periphery 246 and the second end of the periphery 246 may have a same shape. The periphery 246 may define other shapes, such as an ovular, square, or amorphous shape.

In some examples, an opening, such as aperture 250, may be formed through the film layer 206 near the first end 241. In some examples, the periphery 246 may define the aperture 250. In some examples, the aperture 250 may be circular. In other embodiments, the aperture 250 may have other shapes, such as elliptical, square, triangular, or amorphous shapes. In some examples, as shown in Figure 2, the aperture 250 may be concentric with the first end 241. For example, the film layer 206 may have a uniform width between the aperture 250 and the periphery 246 at the first end 241. In some examples, the aperture 250 may not be concentric with the first end 241 or the aperture 250 and the first end 241 may be differently shaped, causing a width between the aperture 250 and the periphery 246 at the first end 241 to be variable.

The film layer 206 may also include a cutaway portion, such as notch 252. In some examples, the periphery 246 may define the notch 252. In some examples, as shown in Figure 2, the notch 252 may extend from the second end 243 of the film layer 206 to the aperture 250. In some examples, the notch 252 may be a slot, extending into the film layer 202 from the second end 243 parallel to the length of the film layer 206 between the first end 241 and the second end 243. In some examples, a portion of the periphery 246 may define the aperture 250 and the notch 252. In some examples, the film layer 206 may include an interior edge or inner perimeter, such as periphery 248. For example, as shown in Figure 2, the periphery 248 may define an opening in the film layer 206, such as aperture 254.

In some examples, the periphery 246 may be substantially geometrically similar to the outer periphery 224 of the embossed layer 204 and/or the periphery 212 of the film layer 202. In some examples, the periphery 246 may be substantially parallel to the outer periphery 224 of the embossed layer 204 and/or the periphery 212 of the film layer 202. In some examples, the periphery 246 may extend past the outer periphery 224 of the embossed layer 204 and/or the periphery 212 of the film layer 202. In some examples, the outline of the aperture 254 defined by the periphery 248 may be substantially geometrically similar to the outline of the inner periphery 226. In some examples, the outline of the aperture 254 defined by the periphery 248 may be substantially parallel to the outline of the inner periphery 226. In some examples, the outline of the aperture 254 defined by the periphery 248 may be substantially coextensive with the outline of the inner periphery 226. In some examples, the periphery 248 may be substantially geometrically similar to the path defined by a cross-section of the second surface 234 of the walled portion 232 of the embossed layer 204 taken in a plane substantially orthogonal to the second surface 234. In some examples, the periphery 248 may be substantially parallel to the path defined by a cross-section of the second surface 234 in a plane substantially orthogonal to the second surface 234. In some examples, the periphery 248 may be substantially coextensive with the path defined by a cross section of the second surface in a plane substantially orthogonal to the second surface 234. In some examples, the periphery 248 may be substantially geometrically similar to the path defined by a cross-section of the second surface 234 taken in a plane substantially parallel to the plane defined by the second surface 222 of the planar portion 218 of the embossed layer 204. In some examples, the periphery 248 may be substantially parallel to the path defined by a cross-section of the second surface 234 taken in a plane substantially parallel to the plane defined by the second surface 222. In some examples, the periphery 248 may be substantially coextensive with the path defined by a cross-section of the second surface 234 taken in a plane substantially parallel to the plane defined by the second surface 222.

In some examples, the film layer 206 may be formed from a material capable of providing a fluid seal between two components and/or two environments. For example, the film layer 206 may be formed from any of the materials previously described with respect to cover 110 or film layer 202. In some examples, the film layer 206 may be a flexible material capable of providing a fluid seal, such as a polymer drape. In some examples, the film layer 206 may include one or more of the following materials: polyurethane; cellulosics; hydrophilic polyamides; polyvinyl alcohol; polyvinyl pyrrolidone; hydrophilic acrylics; silicones; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate; co-polyester; and/or polyether block polyamide copolymers.

In some examples, the film layer 206 may include an attachment device, such as an adhesive. The attachment device can be disposed on some or all of the first surface 242. In some examples, the attachment device of the film layer 206 may be any attachment previously described with respect to the attachment device of the cover 110 or the attachment device of the film layer 202. For example, the attachment device may be a medically-acceptable, pressure sensitive adhesive, an acrylic adhesive, such as an acrylic adhesive with a coating weight of about 25-65 g.s.m., a double-sided tape, a paste, a hydrocolloid, a hydrogel, a silicone gel, or an organogel.

The pressure-offloading component 124 may also include one or more securing tabs 256. In some examples, the securing tabs 256 may be elongated strips formed from any of the materials previously described with respect to the cover 110. In some examples, the securing tabs 256 may have an attachment device, such as an adhesive, disposed on some or all of a surface of the securing tab 256, such as the surface of the securing tab 256 configured to face the second surface 238 of the planar portion 236 of the embossed layer 204.

The pressure-offloading component 124 may further include one or more release liners, such as release liner 258, release liner 260, and/or release liner 262. In some examples, the release liner 258, the release liner 260, and/or the release liner 262 may protect any or all of the adhesives disposed on the first surface 208, the first surface 220, and/or the first surface 242 prior to the application of the pressure-offloading component 124. The release liner 258, the release liner 260, and/or the release liner 262 may also provide stiffness to assist with the deployment of the pressure-offloading component 124. In some examples, the release liner 258, the release liner 260, and/or the release liner 262 may be a casting paper or a film, such as a polyethylene film. In some examples, the release liner 258, the release liner 260, and/or the release liner 262 may be formed from a polyester material, such as polyethylene terephthalate (PET), or a similar polar semi-crystalline polymer. The use of a polar semi-crystalline polymer for the release liner 258, the release liner 260, and/or the release liner 262 may substantially prevent the wrinkling or other deformation of the pressure-offloading component 124. For example, the polar semi-crystalline polymer may be highly orientated and resistant to softening, swelling, or other deformation that may occur when brought into contact with the components of the pressure-offloading component 124, or when subjected to temperature of environmental variations, or sterilization. In some examples, a release agent may be disposed on a side of the release liner 258, the release liner 260, and/or the release liner 262 configured to face any or all of the adhesives disposed on the first surface 208, the first surface 220, and/or the first surface 242. In some examples, the release agent may be a silicone coating and may have a release factor suitable to facilitate the removal of the release liner 258, the release liner 260, and/or the release liner 262 by hand without damaging or deforming the pressure-offloading component 124. In some examples, the release agent may be a fluorocarbon or a fluorosilicone. In some examples, the release liner 258, the release liner 260, and/or the release liner 262 may be uncoated or otherwise used without a release agent.

In some examples, as shown in Figure 2, the release liner 258 may include a contact portion 264 configured to contact any or all of the adhesives disposed on the first surface 208, the first surface 220, and/or the first surface 242, a first handling portion 266 configured to extend away from the pressure-offloading component 124, and/or a second handling portion 268 configured to extend away from the pressure-offloading component 124. In some examples, the release liner 260 may include a contact portion 270 configured to contact any or all of the adhesives disposed on the first surface 208, the first surface 220, and/or the first surface 242, and a handling portion 272 configured to extend away from the pressure-offloading component 124. In some examples, the release liner 262 may include a contact portion 274 configured to contact any or all of the adhesives disposed on the first surface 208, the first surface 220, and/or the first surface 242, and a handling portion 276 configured to extend away from the pressure-offloading component 124.

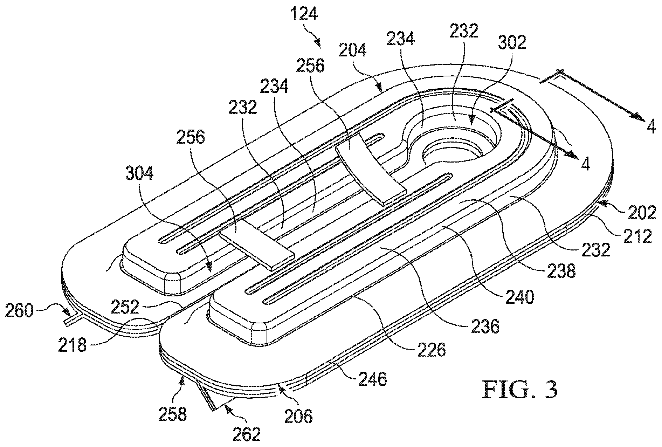

Figure 3 is an isometric view of an assembled example of the pressure-offloading component 124 of Figure 2. As shown in the example of Figure 3, the components may be assembled in a stacked relationship. The embossed layer 204 may be disposed over the film layer 202. In some examples, at least a portion of the first surface 220 (not shown) of the planar portion 218 (not shown) of the embossed layer 204 may be coupled, such as by an adhesive or by welding, to at least a portion of the second surface 210 (not shown) of the film layer 202. The film layer 206 may be disposed over the embossed layer 204 and the film layer 202. In some examples, at least a portion of the first surface 242 (not shown) of the film layer 206 may be coupled, such as by an adhesive or by welding, to at least a portion of the second surface 222 (not shown) of the planar portion 218 and/or to at least a portion of the second surface 210. In some examples, as shown in Figure 3, the walled portion 232 of the embossed layer 204 may be received through the aperture 254 defined by the inner periphery 248 of the film layer 206.

In some examples, the outer periphery 224 of the planar portion 218 of the embossed layer 204 may bound an area smaller than an area bound by the periphery 212 of the of the film layer 202. In some examples, the outer periphery 224 may be contained within the periphery 212. In some examples, the outer periphery 224 may bound an area larger than the periphery 212, and the periphery 212 may be contained within the outer periphery 224. In some examples, the outer periphery 224 may be substantially coextensive with the periphery 212. In some examples, the periphery 248 may be substantially coextensive with the inner inner periphery 226 of the planar portion 218 forming the base of the walled portion 232. In some examples, at least a portion of the periphery 246 of the film layer 206 may be substantially similar to, parallel to, or coextensive with at least a portion of with the periphery 212 of the film layer 206 such that the notch 252 of the film layer 206 may be substantially aligned or coextensive with the notch 230 of the embossed layer 204 and/or the notch 216 of the film layer 202. In some examples, the aperture 250 of the film layer 206 may be substantially aligned or coextensive with the aperture 228 of the embossed layer 204 and/or the aperture 214 of the film layer 202.

In some examples, a portion of the profile of the second surface 234 of the walled portion 232 may define an annular region 302. In some examples, a portion of the second surface of the walled portion 232 taken in a plane parallel to the second surface 244 of the film layer 206, the first surface 242, the second surface 210, and/or the first surface 208 (not shown) may define an annular region 302 substantially geometrically similar to the path define by the aperture 250 and/or the aperture 214. In some examples, a portion of the profile of the second surface 234 taken in a plane parallel to the second surface 244, the first surface 242, the second surface 210, and/or the first surface 208 may define an annular region 302 substantially parallel to the path defined by the aperture 250 and/or the aperture 214.

In some examples, a portion of the profile of the second surface 234 may define a slotted region 304. In some examples, a portion of the profile of the second surface 234 taken in a plane parallel to the second surface 244, the first surface 242, the second surface 210, and/or the first surface 208 may define a slotted region 304 substantially geometrically similar to the path defined by the notch 252 and/or the notch 216. In some examples, a portion of the profile of the second surface 234 taken in a plane parallel to the second surface 244, the first surface 242, the second surface 210, and/or the first surface 208 may define a slotted region 304 substantially parallel to the path defined by the notch 252 and/or the notch 216.

As shown in the example of Figure 3, the one or more securing tabs 256 may be coupled, such as by adhesive, to at least a portion of the second surface 238 of the planar portion 236 of the embossed layer 204. In some examples, the one or more securing tabs 256 may form a bridge over at least a portion of the slotted region 304 and/or the annular region 302.

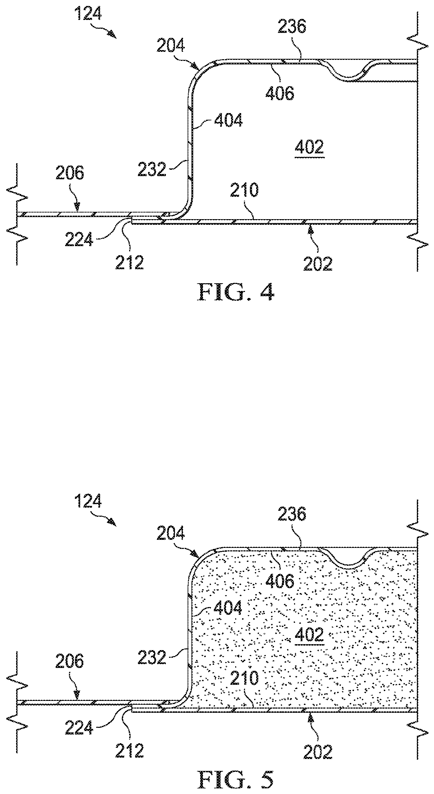

Figure 4 is a cross-sectional view of an example pressure-offloading component 124 of Figure 3, taken at line 4-4, illustrating additional details associated with some embodiments. In some examples, the film layer 202 may be coupled to the embossed layer 204 near the periphery 212 of the film layer 202 to define a cavity or cell, such as interior space 402, between the film layer 202 and the embossed layer 204. In some examples, the film layer 202 and the embossed layer 204 may form a fluid seal between the layers, substantially fluidly isolating the interior space 402 from the external environment. In some examples, the interior space 402 may be defined as the space enclosed by the second surface 210 of the film layer 202, the first surface 404 of the walled portion 232 of the embossed layer 204, and the first surface 406 of the planar portion 236 of the embossed layer 204. In some examples, the interior space 402 may be filled with a gas, such as air from the atmosphere, or an inert gas, such as nitrogen or argon. In some examples, the interior space 402 may be filled with a liquid, such as water or a saline solution.

Figure 5 is a cross-sectional view, illustrating additional details that may be associated with some embodiments of the pressure-offloading component 124 shown in Figure 4. In some examples, the interior space 402 may be filled with a material suitable for distributing the weight of a patient, such as a foam or gel material.

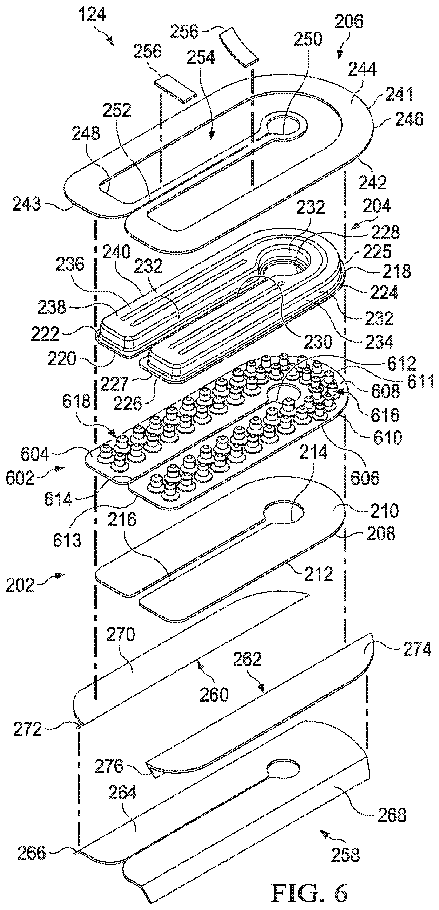

Figure 6 is an exploded view of an example embodiment of the pressure-offloading component 124 of Figure 2 which further includes an additional thermoformed layer. As shown in Figure 6, some examples of the pressure-offloading component 124 may include a second thermoformed layer suitable for forming a cavity or cell, such as embossed layer 602. The embossed layer 602 may be a substantially sheet-like structure with three dimensional features suitable for forming a cavity or a plurality of cavities. When assembled, the embossed layer 602 and the film layer 202 may define a cell or a plurality of cells. In some examples, the embossed layer 602 may have a first end 611 opposite a second end 613. In some examples, the embossed layer 602 may generally define a substantially rectangular shape with the first end 611 and the second end 613 being separated by a length of the embossed layer 602. In some examples, the first end 611 may be substantially semi-circular or substantially curved. In some examples, the second end 613 may be substantially angular or squared-off. In some examples, the first end 611 and the second end 613 may have substantially the same shape. In some examples, the first end 611 and/or the second end 613 may have other shapes, such as ovular, squared, or amorphous shapes.

The embossed layer 602 may have a planar portion 604. The planar portion 604 may include a first surface 606 opposite a second surface 608. In some examples, the first surface 606 may be configured to be oriented to face the tissue site, and the second surface 608 may be configured to face away from the tissue site. The first surface 606 and the second surface 608 may be substantially planar surfaces. In some examples, the first surface 606 and the second surface 608 may be substantially parallel. In some examples, the planar portion 604 may have a substantially uniform thickness between the first surface 606 and the second surface 608. In some examples, the planar portion 604 may have a uniform thickness in a range of between about 250 micrometers to about 3,000 micrometers. For example, the planar portion 604 may have a uniform thickness in a range of about 500 micrometers to about 1,000 micrometers. In some examples, the planar portion 604 may have a uniform thickness in a range of about 800 micrometers to about 3,000 micrometers. In some examples, the planar portion 604 may have a uniform thickness in a range of about 250 micrometers and about 1,000 micrometers. In some examples, the planar portion 604 may have a uniform thickness of about 500 micrometers. The planar portion 604 may have an outer edge or perimeter, such as a periphery 610. The periphery 610 may define an outline or a shape of the planar portion 604.

The embossed layer 602 may include an opening, such as aperture 612 formed near the first end 611. In some examples, the periphery 610 may define the aperture 612. In some examples, the aperture 612 may be circular. In some examples, the aperture 612 may have other shapes, such as elliptical, triangular, rectangular, polyhedral, or amorphous shapes. In some examples, as shown in Figure 6, the aperture 612 may be concentric with the first end 611. For example, the embossed layer 602 may have a uniform width between the aperture 612 and the portion of the periphery 610 defining the first end 611. In some examples, the aperture 612 may not be concentric with the first end 611. In some examples, the aperture 612 may not have a similar shape to the shape of the first end 611. In some examples, the width between the aperture 612 and the portion of the periphery 610 defining the first end 611 may be variable.

The embossed layer 602 may also include a cutaway portion, such as a notch 614. In some examples, the periphery 610 may define the notch 614. In some examples, as shown in Figure 6, the notch 614 may extend from the second end 613 of the planar portion 604 to the aperture 612. In some examples, the notch 614 may be a slot, extending into the planar portion 604 from the second end 613 parallel to the length of the planar portion 604. In some examples, a portion of the periphery 610 may define the aperture 612 and the notch 614.

The embossed layer 602 may further include a first plurality of standoffs formed in the embossed layer 602, such as a plurality of standoffs 616. In some examples, the embossed layer 602 may also include a second plurality of standoffs, such as a plurality of standoffs 618. In some examples, as shown in Figure 6, each standoff of the plurality of standoffs 616 may include a base portion connected to the planar portion 604, and a center portion connecting the base portion to an end portion. In some examples, the base portion of each standoff of the plurality of standoffs 616 may have a substantially spherical segment shape. In some examples, the center portion of each standoff of the plurality of standoffs 616 may be substantially cylindrical in shape. In some examples, the end portion of each standoff of the plurality of standoffs 616 may have a substantially spherical segment shape or a hemispherical shape. In some examples, each standoff of the plurality of standoffs 616 may have a substantially hemispherical, spherical segment, cylindrical, cuboidal, polyhedral or amorphous shape. In some examples, each standoff of the plurality of standoffs 616 may be hollow, and have a same thickness as the thickness of the embossed layer 602. In some examples, each standoff of the plurality of standoffs 616 may be hollow and define a cavity or cell formed in the first surface 606 of the planar portion 604. In some examples, each standoff of the plurality of standoffs 616 may be substantially filled such that the first surface 606 of the planar portion 604 remains a substantially planar surface without cavities or cells.

In some examples, as shown in Figure 6, each standoff of the plurality of standoffs 618 may include a base portion connected to the planar portion 604, and a center portion connecting the base portion to an end portion. In some examples, the base portion of each standoff of the plurality of standoffs 618 may have a substantially spherical segment shape. In some examples, the center portion of each standoff of the plurality of standoffs 618 may be substantially cylindrical in shape. In some examples, the end portion of each standoff of the plurality of standoffs 618 may have a substantially spherical segment shape or a hemispherical shape. In some examples, each standoff of the plurality of standoffs 618 may have a substantially hemispherical, spherical segment, cylindrical, cuboidal, polyhedral or amorphous shape. In some examples, each standoff of the plurality of standoffs 618 may be hollow, and have a same thickness as the thickness of the embossed layer 602. In some examples, each standoff of the plurality of standoffs 618 may be hollow and define a cavity or cell formed in the first surface 606 of the planar portion 604. In some examples, each standoff of the plurality of standoffs 618 may be substantially filled such that the first surface 606 of the planar portion 604 remains a substantially planar surface without cavities or cells.

In some examples, the plurality of standoffs 616 may be arranged in a pattern near the first end 611 of the planar portion 604. In some examples, the plurality of standoffs 616 may be arranged in a pattern of substantially annular rows. In some examples, the plurality of standoffs 616 may be arranged in a pattern of substantially concentric substantially annular rows. In some examples, each row of the plurality of standoffs 616 may be substantially concentric with the aperture 612. In some examples, each row of the standoffs 616 may be substantially concentric with a portion of the periphery 610 defining the curved portion of the first end 611. In some examples, the plurality of standoffs 618 may be arranged in a pattern near the second end 613 of the planar portion 604. In some examples, the plurality of standoffs 618 may be arranged in a pattern of rows. In some examples, the plurality of standoffs 618 may be arranged in a pattern of rows, wherein each row substantially extends along a length of the notch 614. In some examples, the plurality of standoffs 618 may be arranged in a pattern of substantially parallel rows. In some examples, each row of the plurality of standoffs 618 may be substantially parallel with each other row. In some examples, each row of the plurality of standoffs 618 may be substantially parallel with an edge of the notch 614. In some examples, each standoff of the plurality of standoffs 616 may be substantially smaller than each standoff of the plurality of standoffs 618. In some examples, each standoff of the plurality of standoffs 616 may be the substantially the same size as each standoff of the plurality of standoffs 618.

In some examples, the periphery 610 may be substantially geometrically similar to the periphery 212 of the film layer 202 and/or the outer periphery 224 of the planar portion 218 of the embossed layer 204. In some examples, the periphery 610 may be substantially parallel to the periphery to the periphery 212 and/or the outer periphery 224. In some examples, the periphery 610 may be substantially coextensive with one or both of the periphery 212 and/or the outer periphery 224.

In some examples, the embossed layer 602 may be formed from any of the materials previously described with respect to the embossed layer 602. In some examples, the embossed layer 602 may be formed from a non-porous polymeric film which may include any flexible material that can be manipulated to form a defined shape which may enclose a space, including thermoplastics. Non-limiting examples of suitable thermoplastics include polyethylene homopolymers (such as low-density polyethylene (LDPE) or high-density polyethylene (HDPE)) and/or polyethylene copolymers (such as ionomers, EVA, EMA, heterogeneous (Zeigler-Natta catalyzed) ethylene/alpha-olefin copolymers, and homogenous (metallocene, single-site catalyzed) ethylene/alpha olefin copolymers). Ethylene/alphaolefin copolymers are copolymers are copolymers of ethylene with one or more comonomers selected from C₃ to C₂₀ alpha-olefins, such as 1-butene, 1-pentene, 1-hexene, 1-octene, methyl pentene in which the polymer molecules include long chains with relatively few side chain branches. Examples of suitable ethyle/alpha-olefin copolymers include linear low-density polyethylene (LLDPE), linear medium-density polyethylene (LMDPE), very-low-density polyethylene (VLDPE), and ultra-low-density polyethylene (ULDPE). In some examples, the embossed layer 602 may be formed from or include polypropylene homopolymers, polypropylene copolymers, polyesters, polystyrenes, polyamides, and polycarbonates. In some examples, the embossed layer 602 may be formed from an HDPE material with an ultimate tensile strength of about 37 MPa and a yield strength in a range of about 26-33 MPa. In some examples, the embossed layer 204 may be formed from a thermoplastic polyurethane film, such PLATILON^{®} thermoplastic films available from Convestro AG. For example, a thermoplastic polyurethane film having an ultimate tensile strength of about 60 MPa and a yield strength of greater than about 10 MPa may be used.

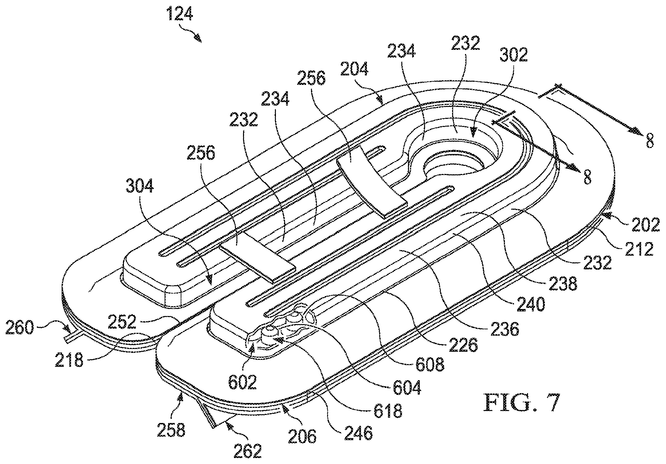

Figure 7 is an isometric cutaway view of an assembled example of the pressure-offloading component 124 of Figure 6 with a portion of the embossed layer 204 removed to show a portion of the embossed layer 602. As shown in the example of Figure 7, the components may be assembled in a stacked relationship. The embossed layer 602 may be disposed over the film layer 202. In some examples, at least a portion of the first surface 606 of the planar portion 604 of the embossed layer 602 may be coupled, such as by an adhesive or welding, to at least a portion of the second surface 210 (not shown) of the film layer 202. The embossed layer 204 may be disposed over the embossed layer 602 and the film layer 202. In some examples, at least a portion of the first surface 220 (not shown) of the planar portion 218 (not shown) of the embossed layer 204 may be coupled, such as by an adhesive or by welding, to at least a portion of the second surface 608 of the planar portion 604 of the embossed layer 602 and/or the second surface 210. The film layer 206 may be disposed over the embossed layer 204, the embossed layer 602, and the film layer 202. In some examples, at least a portion of the first surface 242 (not shown) of the film layer 206 may be coupled, such as by an adhesive or by welding, to at least a portion of the second surface 222 (not shown) of the planar portion 218, to at least a portion of the second surface 608, and/or to at least a portion of the second surface 210.

In some examples, the periphery 610 (not shown) of the planar portion 604 of the embossed layer 602 may bound an area smaller than an area bound by the periphery 212 of the film layer 202. In some examples, the periphery 610 may be contained within the periphery 212. In some examples, the periphery 610 may bound an area larger than the periphery 212, and the periphery 212 may be contained within the periphery 610. In some examples, the periphery 610 may be substantially coextensive with the periphery 212. In some examples, the outer periphery 224 (not shown) of the planar portion 218 may bound an area smaller than an area bound by the periphery 610. In some examples, the outer periphery 224 may be substantially coextensive with the periphery 610. In some example, the outer periphery 224 may bound an area larger than an area bound by the periphery 610.

In some examples, as shown in Figure 7, at least a portion of the periphery 610 may be substantially similar to, parallel to, or coextensive with at least a portion of the periphery 246, outer periphery 224, and/or periphery 212 such that the notch 614 of the embossed layer 602 may be substantially aligned or coextensive with the notch 252 of the film layer 206, the notch 230 of the embossed layer 204, and/or the notch 216 of the film layer 202. In some examples, the aperture 612 of the embossed layer 602 may be substantially aligned or coextensive with the aperture 250 of the film layer 206, the aperture 228 of the embossed layer 204, and/or the aperture 214 of the film layer 202.

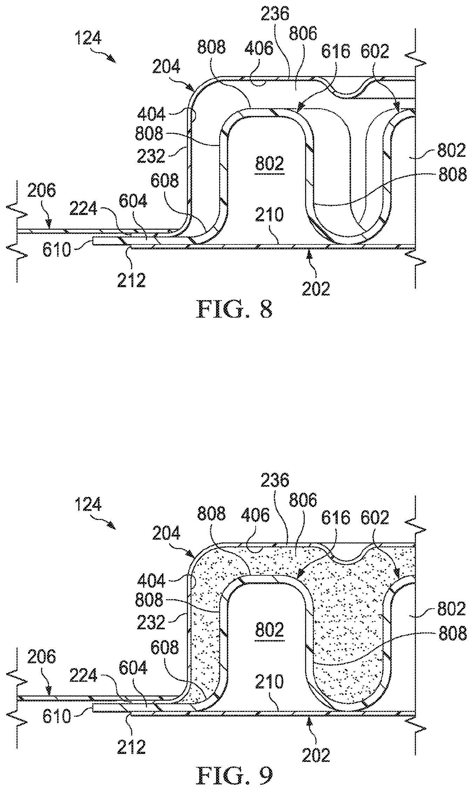

Figure 8 is a cross-sectional view of an example pressure-offloading component 124 of Figure 7, taken at line 8-8, illustrating additional details associated with some embodiments. In some examples, the film layer 202 may be coupled to the embossed layer 602 near the periphery 212 of the film layer 202 to define one or more cavities or cells, such as interior spaces 802, between the film layer 202 and the embossed layer 602. In some examples, the film layer 202 and the embossed layer 602 may form a fluid seal between the layers, substantially isolating the interior spaces 802 from the external environment. In some examples, the interior spaces 802 may be defined as the spaces enclosed by the second surface 210 of the film layer 202 and the interior surfaces 804 of the first plurality of standoffs 616 and/or the interior surfaces of the second plurality of standoffs 618 (not shown). In some examples, the embossed layer 204 may be coupled to the embossed layer 602 near the outer periphery 224 of the embossed layer 204 to define a cavity or cell, such as interior space 806 between the embossed layer 204 and the embossed layer 602. In some examples, the embossed layer 204 and the embossed layer 602 may form a fluid seal between the layers, substantially isolating the interior space 806 from the external environment. In some examples, the interior space 806 may be defined as the space enclosed by a portion of the second surface 608 of the planar portion 604 of the embossed layer 602, the exterior surfaces 808 of the first plurality of standoffs 616 and/or the exterior surfaces of the second plurality of standoffs 618 (not shown), the first surface 404 of the walled portion 232 of the embossed layer 204, and the first surface 406 of the planar portion 236 of the embossed layer 204. In some examples, the interior spaces 802 may be filled with a gas, such as air from the atmosphere, or an inert gas, such as nitrogen or argon. In some examples, the interior spaces 802 may be filled with a liquid, such as water or a saline solution. In some examples, the interior space 806 may be filled with a gas, such as air from the atmosphere, or an inert gas, such as nitrogen or argon. In some examples, the interior space 806 may be filled with a liquid, such as water or a saline solution.

Figure 9 is a cross-sectional view, illustrating additional details that may be associated with some embodiments of the pressure-offloading component 124 shown in Figure 4. As shown in Figure 9, the interior space 806 may be filled with a material suitable for distributing the weight of a patient, such as a foam or gel material, while the interior space 802 may be filled with a liquid or a gas. In some examples (not shown), the interior space 802 may be filled with a material suitable for distributing the weight of a patient, such as a foam or gel material, while the interior space 806 may be filled with a liquid or a gas. In some examples (not shown), the interior space 802 and the interior space 806 may be filled with a material suitable for distributing the weight of a patient, such as a foam or gel material.

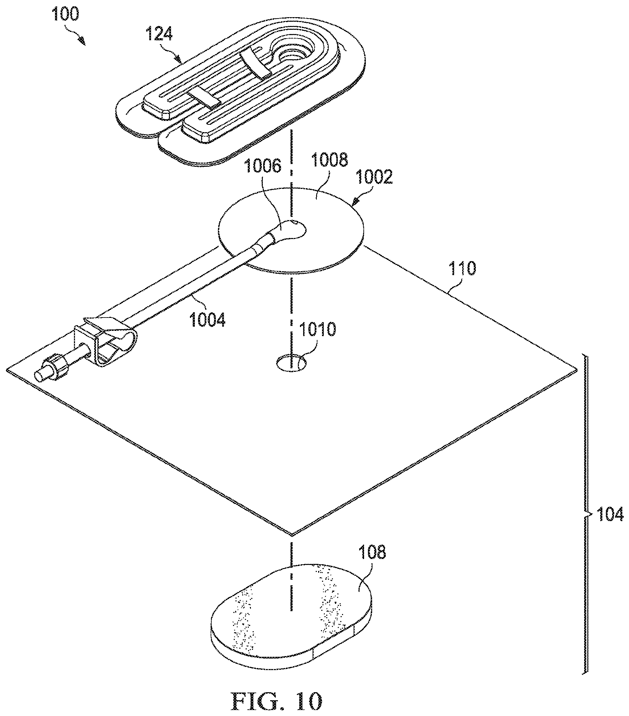

Figure 10 is an exploded view of an example embodiment of a therapy system 100 of Figure 1 including a pressure-offloading component 124 in accordance with this specification. In some embodiments, the system 100 may include a dressing interface as previously described, such as dressing interface 1002, and a fluid conductor as previously described, such as tube 1004. In some examples, the dressing interface 1002 may include an elbow connector 1006 configured to be fluidly coupled to the tube 1004. In some examples, the dressing interface 1002 may include a drape 1008 with an adhesive disposed on a side of the drape 1008 configured to face the cover 110. The drape 1008 may be configured to couple or adhere the elbow connector 1006 to the cover 110. The cover 110 may be configured to be disposed over a tissue interface as previously described, such as tissue interface 108. The tissue interface 108 may be configured to be in fluid communication with the tube 1004 through the elbow connector 1006 and an aperture 1010 formed through the cover 110.

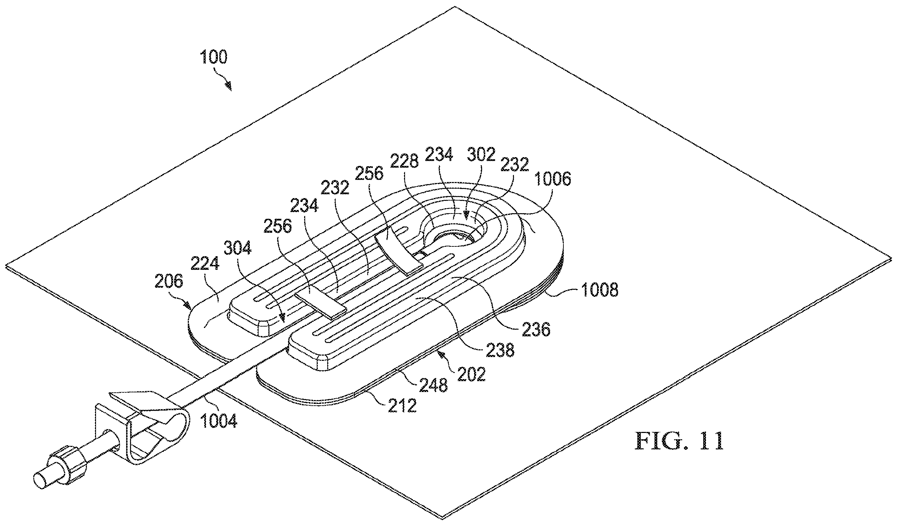

Figure 11 is an isometric view of an assembled example of the therapy system 100 of Figure 10. As shown in Figure 11, the cover 110 may be disposed over the tissue interface 108 (not shown), and the elbow connector 1006 may be coupled to the cover 110, such as with the drape 1008 as previously described. The tube 1004 may be coupled to the elbow connector 1006. The pressure-offloading component 124 may be coupled to the cover 110. In some examples, at least a portion of the first surface 208 (not shown) of the film layer 202 of the pressure-offloading component 124 may be coupled, such as adhered using an adhesive disposed on the first surface 208, to the cover 110. As shown in Figure 11, the elbow connector 1006 may be received through the aperture 214 (not shown) of the film layer 202 and the aperture 228 of the embossed layer 204. In some examples, the elbow connector 1006 may be disposed in the annular region 302 between a portion of the second surface 234 of the walled portion 232 of the embossed layer 204 at the first end. At least a portion of the tube 1004 may be received through the notch 216 (not shown) of the film layer 202, the notch 230 (not shown) of the embossed layer 204, and the notch 252 (not shown) of the film layer 206 to be disposed in the slotted region 304 between a portion of the second surface 234 of the walled portion 232 of the embossed layer 204 at the second end. In some examples, as shown in Figure 11, the notch 216, the notch 230, and/or the notch 252 may be narrower than a diameter of the tube 1004, and the tube 1004 may be disposed on top of the second surface 244 of the film layer 206 and between two portions of the second surface 234 at the slotted region 304. In some examples, the notch 216, the notch 230, and the notch 252 may be wider than a diameter of the tube 1004, and the tube 1004 may be disposed on the cover 110 between the portions of the periphery 212 of the film layer 202 defining the notch 216, the portions of the outer periphery 224 (not shown) defining the notch 230, the portions of the periphery 248 defining the notch 252, and between two portions of the second surface 234 at the slotted region 304.

In some examples, a height of the elbow connector 1006 and a height of the tube 1004 may be less than a height of the second surface 238 of the planar portion 236 of the embossed layer 204 in a direction normal to the surface of the cover 110 on which the pressure-offloading component 124 is disposed. The one or more securing tabs 256 may be coupled to a portion of the second surface 238 to be disposed over the tube 1004 and/or the elbow connector 1006, substantially securing the tube 1004 relative to the pressure-offloading component 124.

The systems, apparatuses, and methods described herein may provide significant advantages. For example, in some embodiments of the system 100 where the height of the second surface 238 of the planar portion 236 of the embossed layer 204 is greater than a height of the elbow connector 1006 and a height of the tube 1004 in a direction normal to the surface of the cover 110 on which the pressure-offloading component 124 is disposed, the relatively large second surface 238 may come into contact with an opposing surface, such as a surface of a bed. The normal force resulting from the contact of the second surface 238 with the opposing surface will be distributed across a relatively large area of the second surface 238, lowering the pressure exerted by the pressure-offloading component 124 on the cover 110, and lowering the force exerted by the pressure-offloading component 124 on the cover 110 at any given point. In examples of the system 100 without the pressure-offloading component 124, the normal force resulting from the contact of the relatively small surface areas of the tube 1004 and the elbow connector 1006 will be distributed across a relatively small surface area of the tube 1004 and elbow connector 1006 in a plane substantially parallel with the surface of the cover 110 on which the tube 1004 and elbow connector 1006 are disposed, resulting in an increased pressure exerted by the tube 1004 and/or the elbow connector 1006 on the cover 110 and an increased force exerted by the tube 1004 and/or the elbow connector 1006 on the cover 110 at any given point in comparison to examples of the system 100 including the pressure-offloading component 124. By including a pressure-offloading component 124 to reduce the pressure and force exerted on the cover 110 at any given point or area, the overall comfort of a patient undergoing negative-pressure wound therapy may be increased, particularly where the patient is undergoing negative-pressure wound therapy in a region of the body which may experience prolonged contact with another surface.

Additionally or alternatively, by including a conformable material such as a liquid, foam, and/or gel in the interior space 402, the interior spaces 802, and/or the interior space 806 as previously described, the overall ability of the pressure-offloading component 124 to resist deformation in a direction substantially normal to the second surface 238 may be increased, increasing the force that may be distributed across the pressure-offloading component 124 before the height of the pressure-offloading component 124 in a direction normal to the surface of the cover 110 may be reduced to become equal with the height of the tube 1004 and/or the elbow connector in the direction normal to the surface of the cover 110.

Additionally or alternatively, by providing a central release liner, such as release liner 258, and one or more release liners at the edges of the pressure-offloading component 124, such as release liner 260 and/or release liner 262, the release liners may be used to aid the user in easily and precisely positioning the pressure-offloading component 124 on the cover 110. For example, the user may initially remove the central release liner 258, while leaving the release liner 260 and the release liner 262 attached to the pressure-offloading component 124. In such a configuration, only the adhesive disposed on central portions of the first surface 208 of the film layer 202 and/or the first surface 242 of the film layer 206 may be exposed. The user may handle or maneuver the pressure-offloading component 124 without contacting or compromising any of the adhesive by grasping the release liner 260 and/or the release liner 262, precisely placing the pressure-offloading component 124 against the desired surface. Once the pressure-offloading component 124 is in place, the release liner 260 and/or the release liner 262 may be removed, exposing the additional adhesive, which may more securely adhere the pressure-offloading component 124 to the desired surface.

While shown in a few illustrative embodiments, a person having ordinary skill in the art will recognize that the systems, apparatuses, and methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may also be combined or eliminated in various configurations for purposes of sale, manufacture, assembly, or use. For example, in some configurations that dressing 104, container 106, or both may be eliminated or separated from other components for manufacture or sale. In other example configurations, the controller 112 may also be manufactured, configured, assembled, or sold independently of other components.

## Claims

1. An apparatus for distributing force across a tissue site, comprising:
a first film layer (202) having a first end (207) and a second end (209), the first film layer (202) having an aperture (214) near the first end (207) and a notch (216) extending from the second end (209) to the aperture (214); and
an embossed layer (204) coupled to the first film layer (202) near a periphery of the first film layer (202) to define an interior space (402);
wherein the first film layer (202) and the embossed layer (204) form a fluid seal between those layers (202, 204) to fluidly isolate the interior space (402) from the external environment.

2. The apparatus of claim 1, wherein the embossed layer comprises:
a first planar portion (218) having an outer periphery (224) parallel to the periphery of the film layer (202) and an inner periphery (226) parallel to the outer periphery (224);
a walled portion (232) having a base and a top, the base following a first path defined by the inner periphery (226), the top following a second path parallel to the first path; and
a second planar portion (236) having a periphery (240) defined by the second path.

3. The apparatus of claim 2, wherein the interior space (402) is defined by the first film layer (202), the walled portion (232), and the second planar portion (236).

4. The apparatus of any of claims 2-3, wherein the walled portion (232) is orthogonal to the first planar portion (218) and/or the second planar portion (236).

5. The apparatus of any of claims 1-4, further comprising a foam disposed in the interior space (402).

6. The apparatus of any of claims 2-5, further comprising a second film layer (602) coupled to the embossed layer (204).

7. The apparatus of claim 6, wherein the second film layer (602) is coupled to the embossed layer (204) at the first planar portion (218).

8. The apparatus of claim 7, wherein the second film layer (602) includes a first end and a second end, an aperture near the first end, and a notch extending from the second end to the aperture of the second film layer (602).

9. The apparatus of claim 8, wherein the second film layer (602) comprises a second aperture.

10. The apparatus of claim 9, wherein the second aperture has a periphery parallel to the inner periphery (226) of the first planar portion (218).

11. The apparatus of claim 10, wherein the periphery of the second aperture is coextensive with the inner periphery (226) of the first planar portion (218).

12. The apparatus of any of claims 1-11, wherein the notch (216) of the first film layer (202) is linear.

13. The apparatus of any of claims 8-12, wherein the aperture of the second film layer (602) is annular.

14. The apparatus of any of claims 8-13, wherein the notch of the second film layer (602) is linear.

15. The apparatus of any of claims 1-14, further comprising a first adhesive disposed on a surface of the first film layer (202) opposite the embossed layer (204).

## Patentansprüche

1. Eine Vorrichtung zum Verteilen von Kraft über eine Gewebestelle, aufweisend:
eine erste Folienschicht (202), die ein erstes Ende (207) und ein zweites Ende (209) hat, wobei die erste Folienschicht (202) eine Öffnung (214) in der Nähe des ersten Endes (207) und eine Kerbe (216), die sich von dem zweiten Ende (209) zu der Öffnung (214) erstreckt, hat; und
eine geprägte Schicht (204), die mit der ersten Folienschicht (202) in der Nähe eines Umfangs der ersten Folienschicht (202) gekoppelt ist, um einen Innenraum (402) zu definieren;
wobei die erste Folienschicht (202) und die geprägte Schicht (204) eine Fluiddichtung zwischen diesen Schichten (202, 204) ausbilden, um den Innenraum (402) von der äußeren Umgebung fluidisch zu isolieren.

2. Die Vorrichtung nach Anspruch 1, wobei die geprägte Schicht aufweist:
einen ersten ebenen Abschnitt (218), der einen äußeren Umfang (224) parallel zu dem Umfang der Folienschicht (202) und einen inneren Umfang (226) parallel zu dem äußeren Umfang (224) hat;
einen Wandabschnitt (232), der eine Basis und eine Oberseite hat, wobei die Basis einem ersten Pfad folgt, der durch den inneren Umfang (226) definiert ist, und die Oberseite einem zweiten Pfad folgt, der parallel zu dem ersten Pfad ist; und
einen zweiten ebenen Abschnitt (236), der einen Umfang (240) hat, der durch den zweiten Pfad definiert ist.

3. Die Vorrichtung nach Anspruch 2, wobei der Innenraum (402) durch die erste Folienschicht (202), den Wandabschnitt (232) und den zweiten ebenen Abschnitt (236) definiert ist.

4. Die Vorrichtung nach einem der Ansprüche 2 bis 3, wobei der Wandabschnitt (232) orthogonal zu dem ersten ebenen Abschnitt (218) und/oder zu dem zweiten ebenen Abschnitt (236) ist.

5. Die Vorrichtung nach einem der Ansprüche 1 bis 4, ferner aufweisend einen in dem Innenraum (402) angeordneten Schaum.

6. Die Vorrichtung nach einem der Ansprüche 2 bis 5, ferner aufweisend eine zweite Folienschicht (602), die mit der geprägten Schicht (204) gekoppelt ist.

7. Die Vorrichtung nach Anspruch 6, wobei die zweite Folienschicht (602) an dem ersten ebenen Abschnitt (218) mit der geprägten Schicht (204) gekoppelt ist.

8. Die Vorrichtung nach Anspruch 7, wobei die zweite Folienschicht (602) ein erstes und ein zweites Ende, eine Öffnung in der Nähe des ersten Endes und eine Kerbe einschließt, die sich von dem zweiten Ende zu der Öffnung der zweiten Folienschicht (602) erstreckt.

9. Die Vorrichtung nach Anspruch 8, wobei die zweite Folienschicht (602) eine zweite Öffnung aufweist.

10. Die Vorrichtung nach Anspruch 9, wobei die zweite Öffnung einen Umfang parallel zu dem inneren Umfang (226) des ersten ebenen Abschnitts (218) hat.

11. Die Vorrichtung nach Anspruch 10, wobei der Umfang der zweiten Öffnung mit dem inneren Umfang (226) des ersten ebenen Abschnitts (218) deckungsgleich ist.

12. Die Vorrichtung nach einem der Ansprüche 1 bis 11, wobei die Kerbe (216) der ersten Folienschicht (202) linear ist.

13. Die Vorrichtung nach einem der Ansprüche 8 bis 12, wobei die Öffnung der zweiten Folienschicht (602) ringförmig ist.

14. Die Vorrichtung nach einem der Ansprüche 8 bis 13, wobei die Kerbe der zweiten Folienschicht (602) linear ist.

15. Die Vorrichtung nach einem der Ansprüche 1 bis 14, ferner aufweisend einen ersten Klebstoff, der auf einer Oberfläche der ersten Folienschicht (202) gegenüber der geprägten Schicht (204) angeordnet ist.

## Revendications

1. Appareil permettant de distribuer une force à travers un site tissulaire, comprenant :
une première couche de film (202) ayant une première extrémité (207) et une seconde extrémité (209), la première couche de film (202) ayant une ouverture (214) à proximité de la première extrémité (207) et une encoche (216) s'étendant de la seconde extrémité (209) à l'ouverture (214) ; et
une couche gaufrée (204) accouplée à la première couche de film (202) près d'une périphérie de la première couche de film (202) pour définir un espace intérieur (402) ;
dans lequel la première couche de film (202) et la couche gaufrée (204) forment un joint d'étanchéité entre ces couches (202, 204) afin d'isoler fluidiquement l'espace intérieur (402) de l'environnement extérieur.

2. Appareil selon la revendication 1, dans lequel la couche gaufrée comprend :
une première partie plane (218) ayant une périphérie externe (224) parallèle à la périphérie de la couche de film (202) et une périphérie interne (226) parallèle à la périphérie externe (224) ;
une partie à parois (232) ayant une base et un sommet, la base suivant une première trajectoire définie par la périphérie interne (226), le sommet suivant une seconde trajectoire parallèle à la première ; et
une seconde partie plane (236) ayant une périphérie (240) définie par la seconde trajectoire.

3. Appareil selon la revendication 2, dans lequel l'espace intérieur (402) est défini par la première couche de film (202), la partie à parois (232) et la seconde partie plane (236).

4. Appareil selon l'une quelconque des revendications 2 à 3, dans lequel la partie à parois (232) est orthogonale à la première partie plane (218) et/ou à la seconde partie plane (236).

5. Appareil selon l'une quelconque des revendications 1 à 4, comprenant en outre une mousse disposée dans l'espace intérieur (402).

6. Appareil selon l'une quelconque des revendications 2 à 5, comprenant en outre une seconde couche de film (602) accouplée à la couche gaufrée (204).

7. Appareil selon la revendication 6, dans lequel la seconde couche de film (602) est accouplée à la couche gaufrée (204) au niveau de la première partie plane (218).

8. Appareil selon la revendication 7, dans lequel la seconde couche de film (602) comporte une première extrémité et une seconde extrémité, une ouverture à proximité de la première extrémité, et une encoche s'étendant de la seconde extrémité à l'ouverture de la seconde couche de film (602).

9. Appareil selon la revendication 8, dans lequel la seconde couche de film (602) comprend une seconde ouverture.

10. Appareil selon la revendication 9, dans lequel la seconde ouverture a une périphérie parallèle à la périphérie interne (226) de la première partie plane (218).

11. Appareil selon la revendication 10, dans lequel la périphérie de la seconde ouverture est coextensive avec la périphérie interne (226) de la première partie plane (218).

12. Appareil selon l'une quelconque des revendications 1 à 11, dans lequel l'encoche (216) de la première couche de film (202) est linéaire.

13. Appareil selon l'une quelconque des revendications 8 à 12, dans lequel l'ouverture de la seconde couche de film (602) est annulaire.

14. Appareil selon l'une quelconque des revendications 8 à 13, dans lequel l'encoche de la seconde couche de film (602) est linéaire.

15. Appareil selon l'une quelconque des revendications 1 à 14, comprenant en outre un premier adhésif disposé sur une surface de la première couche de film (202) à l'opposé de la couche gaufrée (204).
